# EUROPEAN PATENT APPLICATION

(11) **EP 1 891 972 A1**
(43) Date of publication of application: **27.02.2008**
(21) Application number: 06732290.9
(22) Date of filing: 24.04.2006
(51) Int. Cl.: A61K 45/00, A61K 39/395, A61P 1/16, A61P 3/10

(54) **AGENT FOR PROMOTING HEPATIC CELL REPLICATION AND AGENT FOR IMPROVING INSULIN RESISTANCE**

(30) Priority: 26.04.2005 JP 2005128345
(71) Applicant: Stelic Institute of Regenerative Medicine, Minato-ku Tokyo 1060044 (JP)
(72) Inventor: YONEYAMA, Hiroyuki, Stelic Institute of, Minato-ku, Tokyo 1060044 (JP); ICHIDA, Takafumi, Minato-ku, Tokyo 1080074 (JP); NARUMI, Shosaku, Shinjuku-ku, Tokyo 1600016 (JP)
(74) Representative: Lord, Hilton David
(86) International application number: PCT/JP2006/308580
(87) International publication number: WO 2006/118085

(57) **Abstract**

The present invention aims at providing a medicament and a method for promoting replication of hepatocytes and a medicament and a method for ameliorating insulin resistance. An effective amount of a neutralizing agent for CXCL10 belonging to a subfamily of chemokines which are heparin-binding proteins is administered to the hepatocytes to promote the replication of the hepatocytes. As the neutralizing agent for CXCL10, a factor which is specifically bound to CXCL10 and inhibits an activity of CXCL10 or a factor which inhibits CXCL10 expression is suitably used. By administering the neutralizing agent to impaired hepatic tissue, it is possible to restore and regenerate the hepatic tissue. Meanwhile, by administering an effective amount of the neutralizing agent for CXCL10 to the hepatocytes, the insulin resistance in type II diabetes and metabolic syndrome is ameliorated.

## Description

### TECHNICAL FIELD

The present invention relates to a medicament for promoting replication of hepatocytes, and particularly relates to a hepatocyte replication promoter capable of contributing to regeneration or restoration of the hepatocytes in hepatic tissue having a disorder and a method for promoting proliferation of the hepatocytes using this promoter. Also, the present invention relates to a medicament for ameliorating insulin resistance, and particularly relates to an insulin resistance ameliorating agent capable of ameliorating the insulin resistance and contributing to normalization of glucose tolerance in patients exhibiting the insulin resistance, and a method for ameliorating the insulin resistance using this.

### BACKGROUND ART

Liver is an important organ to perform amino acid metabolism, ammonia metabolism and detoxification of chemicals, and hepatocytes primarily bears such functions. Therefore, hepatic damage due to a trauma or a poison or hepatic dysfunction due to a disease may lead to a life-threatening severe state for a living body. In particular, the patient with acute hepatic failure due to a hepatocyte disorder has a high mortality, and it is estimated that a survival rate when only conservative treatment including plasmapheresis is given is 30% or less. No definitive conservative therapy has been available until now, and liver transplantation is thought to be an only established therapy to enhance the survival rate. However, problems such as long term use of immunosuppressants, risk of infection, high cost, donor shortage and unknown side effects are not solved yet, and the transplantation has a shortcoming that QOL (quality of life) is remarkably diminished. From these, the therapy based on hepatic regeneration has been actively developed as a method better than the liver transplantation in recent years. The liver has been known as the organ having a high regeneration capacity from a long time ago, and mechanisms for proliferation and differentiation of the hepatocytes have been being studied in detail in connection with IL-6 (interleukin 6) and HGF (hepatocyte growth factor). Nonetheless, a factor which initially induces the regeneration of hepatocytes actually in *vivo,* construction of hepatic cords in a regeneration process and a factor which keeps the functions remain yet as large questions (Non-patent documents 1 and 2).

According to the dominative hypothesis, most of the normal hepatocytes are in resting phase, the hepatocytes synthesizing DNA are several percents or less and physiological turnover rounds once in every several years. However, the turnover is accelerated when damaged, and for example it has been proved that the liver is restored in one week in the case of 70% hepatectomy. As a supply source of the hepatocytes newly generated, four supply sources are presumed at present. They are anatomically mature hepatocytes present in a sinusoid region, small hepatocytes in peri-Hering duct in a portal vein region (cluster referred to as oval cells and thought to be hepatic tissue stem cells), bile duct epithelial cells, and cells derived from bone marrow. It is a basic study task for controlling the hepatic regeneration to know origins, contributions and interactions of these cell population, but they have not been elucidated sufficiently yet.

The most clinically important factor for keeping the hepatic functions upon disorder is how to prevent cholestasis, i.e., how to establish the bile excretion mechanism. This is the critical neck for developing an artificial auxiliary liver in recent years. The bile excretion mechanism is not as a single hepatocyte level but as a three dimensional construction of hepatic cords, which makes a problem. In this respect, the manner of the hepatocytes migration has an important significance. At present, two hypotheses are proposed for explaining the differentiation-dependent migration of the hepatocytes in the hepatic regeneration. One of them is a "streaming theory" according to which the hepatic tissue stem cells in the portal vein region migrate from the sinusoid to a central vein region along with the differentiation. The other is a "mosaic theory" according to which the cells constituting one piece randomly migrate while forming a cluster along with the differentiation. The "mosaic theory" is currently dominative, although has not been completely proven yet (Non-patent document 3). Furthermore, there is almost no report on the factor which determines the arrangement of the hepatocytes and how the stem cells involve as the supply source.

In the current development of the hepatic regeneration therapy, there are two mainstream methods: a regeneration promoting therapy using growth factors and cytokines and a method using the stem cells. In the former, TNF-α (tumor necrosis factor), uPA (urokinase type plasminogen activating factor), plasminogen and VEGF (vascular endothelial cell growth factor) in addition to IL-6 and HGF have been studied as candidates of the factors which stimulate the proliferation of hepatocytes. However, they are problematic in sort half life and side effects attributed to systematic action. Crucially, the action thereof in arrangement formation of hepatic cords is unknown. The mainstream method in the latter is a method of engineering embryonic stem cells (ES cells), bone marrow stem cells or tissue stem cells in vitro and returning them *in vivo.* However, there are hurdles which are quality maintenance of the source, an ethical problem and a risk of malignant alteration in a long period of time. Again crucially, the manner of involvement of the stem cells in the formation of the hepatic cords in liver in *vivo* is completely unknown (Non-patent document 4).

A chemokine is an inclusive term of basic and heparin-binding proteins produced in *vivo* which stimulates chemotaxis and activation of leukocyte. The chemokine has four cysteine residues in positions conserved in a primary structure, and is classified into 4 subfamilies, i.e., CXC, CC, C and CX3C by the positions of first two cysteine residues. For their receptors, 19 types such as CXCR1 to 6, CCR1 to 10, CR1 to 2 and CX3CR1 have been reported until now. Each receptor is specifically expressed on particular cells. The chemokine and the chemokine receptor control the chemotaxis and settlement of immune cells to a particular location. For example, although three types of chemokines CXCL9, CXCL10 and CXCL11 have been reported as ligands for the receptor CXCR3, it has been demonstrated that the actions of these three chemokines are not always identical. Therefore, the individual chemokine belonging to the same subfamily has extremely diversified roles, and thus, it is impossible to collectively address them. It is not sufficiently elucidated how the chemokine actually plays the role in *vivo.*

It has been reported that a plurality of chemokines and chemokine receptors are expressed on the hepatocytes and the vile duct epithelial cells, and it has been speculated that they control infiltration of the immune cells upon inflammation (Non-patent document 5). However, it is completely unknown how they are involved in the replication of the hepatocytes and the differentiation dependent migration of the hepatocytes.

It is being demonstrated that CXCR3 which is the chemokine receptor and its ligand CXCL10 are molecules involved in chemotaxis of activated T cells, particularly T helper 1 (Th1) cells and play the roles for so-called Th1 predominant diseases such as infection with virus or bacteria, graft rejection reaction and autoimmune diseases (Non-patent documents 6, 7, 8, 9 and 10). It has been reported that the expression of CXCL10 mRNA is augmented in the various Th1 predominant diseases including viral hepatitis. However, no inhibitory experiment using a neutralizing agent for CXCL10 has been performed in the actual hepatic disorder. Thus, it is still unknown whether its expression is preferable or conversely not preferable *in vivo* in terms of the significance of its expression. In addition, concerning whether its expression acts upon not only the Th1 cell but also the hepatocyte itself, no experimental evidence is available, and it is even scarcely supposed.

Meanwhile, diabetes is a disease caused by relative shortage of insulin which is a hormone having a blood sugar lowering effect. Diabetes is classified into an insulin dependent type I (IDDM) and a non-insulin-dependent type II (NIDDM). Type I diabetes (IDDM) refers to a state where pancreatic β cells which produce insulin have been congenitally destroyed due to autoimmune diseases and the like. On the contrary, type II diabetes is an insulin independent diabetes caused by the combination of environmental factors such as obesity and shortage of exercise and genetic factors. According to the reports from a surveillance study group of Health and Welfare Ministry, a prevalence rate of non-insulin-dependent diabetes (type II, NIDDM) is about 10% in the population of 40 years old or older and the number of patients has reached about 6 millions in Japan. Changes of lifestyle such as Western dietary habits and the shortage of exercise and aging of the population accelerate the increase of diabetes, which is predicted to grow steadily,in the future. A dietetic therapy and a therapeutic exercise form the basic treatments of diabetes. In a pharmacologic therapy, oral antidiabetic drugs such as insulin related enzymes, nateglinide targeting an insulin receptor, α-glucosidase inhibitors, sulfonyl urea (SU) agents, biagnide (BG) agents and thiazolidine based drugs are clinically used. However, the mechanisms involved in insulin resistance are diversified, and it is yet difficult to crucially ameliorate the insulin resistance (Non-patent documents 11, 12, 13, 14 and 15). Lifestyle related diseases including type II diabetes have been collectively being referred to as an inclusive term "metabolic syndrome". These diseases have been actively researched in recent years as "21 century diseases". Since it has been pointed out that there are numerous potential patients, necessity to rapidly provide means for treating them and ameliorating their symptoms has been regarded as important.

The liver takes glucose in from blood and releases glucose into blood, i.e., is an important organ to control blood sugar levels. Insulin converts glucose into glycogen (promotion of glycogen synthesis) by acting upon an insulin receptor expressed on the hepatocytes to store glycogen in the liver and inhibit the release of glucose (gluconeogenesis) into blood. By this mechanism, glucose in blood is taken in the liver (Non-patent document 16). Therefore, lowering the reaction of liver to insulin is an extremely important cause to induce the insulin resistance (Nonpatent documents 17 and 18).

Nonpatent Literature 1: Updated Review; Fausti N., Hepatology 39:1477, 2004.
Nonpatent Literature 2: Taub R., Nature Reviews Molecular Cell Biology 5:836, 2004.
Nonpatent Literature 3: Zajicek G., Am. J. Pathol. 146:772, 1995.
Nonpatent Literature 4: Updated Review; Taub R., Nature Reviews Molecular Cell Biology 5:836, 2004.
Nonpatent Literature 5: Review; Simpson KJ, et al., Clin. Sci. 104:47, 2003.
Nonpatent Literature 6: Khan IA et al., Immunity 12:483, 2000
Nonpatent Literature 7: Liu MT et al., J. Immunol. 166:1750, 2001.
Nonpatent Literature 8: Hancock WW et al., J. Exp. Med. 192:1515, 2000.
Nonpatent Literature 9: Hancock WW et al., J. Exp. Med. 193:975, 2001.
Nonpatent Literature 10: Narumi S et al., Eur. J. Immunol. 32:1784, 2002.
Nonpatent Literature 11: Fujita, T et al., Biochemical Pharmacology (1996) 52 407-411.
Nonpatent Literature 12: Tsukuda, K et al., Horm Metab Res (1998) 30 42-49.
Nonpatent Literature 13: Fujitani, S et al., Metabolism (1996) 45 184-189.
Nonpatent Literature 14: Spiegelman et al., J Clin Invest (1997) 100 1863-1869.
Nonpatent Literature 15: Olefsky et al., Diabetes (1997) 46 1678-1683.
Nonpatent Literature 16: Cherrington AD., Diabetes 48:1198-1214, 1999.
Nonpatent Literature 17: DeFronzo RA., Diabetes rev 5:177-269,1997.
Nonpatent Literature 18: Michael MD et al., Mol. Cell 6:87-97,2000.

### DISCLOSURE OF INVENTION

### PROBLEM TO BE SOLVED BY THE INVENTION

As described above, in order to restore impaired hepatocytes and their dysfunction associated with hepatic tissue damage, another therapeutic method which can be an alternative of or concomitantly used with a transplantation therapy and a cell therapy is required. Under such a circumstance, it is an object of the present invention to provide a medicament to proliferate the hepatocytes and a method of proliferating the hepatocytes by the use thereof. It is another object of the present invention to provide a medicament which is capable of replacing conventional antidiabetic drugs, is also useful for metabolic syndrome and ameliorates insulin resistance of the hepatocytes, and a method for ameliorating the insulin resistance by the use thereof.

### MEANS FOR SOLVING PROBLEM

As a result of an extensive study for solving the aforementioned problems, the present inventors have obtained a finding that CXCL10 which is a ligand for chemokine receptor CXCR3 and a protein classified into the chemokine subfamily CXC is largely involved in regeneration of the hepatocyte. The present inventors advanced the study on CXCL10, and have found that CXCL10 inhibits replication of the hepatocyte. Then the present inventors have obtained an idea that the replication of the hepatocyte can be promoted by inhibiting the function of CXCL10. Then, the present inventors have demonstrated that a neutralizing agent which inhibits the function of CXCL10 actually promotes the replication of the hepatocytes, and have completed the present invention. As realized embodiments, the present invention provides the hepatocyte replication promoter and method for promoting the proliferation of hepatocytes as will be described below.

Furthermore, the present inventors have proven that, in individuals exhibiting the insulin resistance, the neutralizing agent for CXCL10 not only promotes the replication of the hepatocytes but also significantly enhances reactivity and sensitivity of the hepatocyte to insulin, the action mechanism thereof is speculated to be common. It has also been proven that the neutralizing agent for CXCL10 significantly ameliorates abnormal glucose tolerance. From these findings, the present inventors have found out that the neutralizing agent for CXCL10 is also useful as an insulin resistance ameliorating agent based on a quite new mechanism that the neutralizing agent acts upon the hepatocytes to enhance the reactivity to insulin, and have completed the present invention. The present invention thus provides an insulin resistance ameliorating agent and a method for ameliorating the insulin resistance as realized embodiments, which is as follows:

[1] A hepatocyte replication promoter containing a neutralizing agent for CXCL10 which is one of chemokines as an active component. More specifically, the hepatocyte replication promoter may comprise the neutralizing agent for CXCL10 which is one of the chemokines, and a pharmaceutically acceptable carrier.
[2] The hepatocyte replication promoter according to [1] above wherein the replication of at least mature hepatocytes is promoted.
[3] The hepatocyte replication promoter according to [1] or [2] above wherein the replication of the hepatocyte in impaired hepatic tissue is promoted.
[4] The hepatocyte replication promoter according to any one of [1] to [3] above wherein the impairment is selected from the group consisting of an impairment caused by a toxic substance, an impairment by physical damage and an impairment caused by a pathogen.
[5] The hepatocyte replication promoter according to [4] above wherein the impairment caused by the toxic substance is an impairment caused by a disease selected from the group consisting of alcohol induced hepatitis and drug induced hepatic disorders.
[6] The hepatocyte replication promoter according to [4] above wherein the pathogen is hepatitis virus.
[7] The hepatocyte replication promoter according to [4] above wherein the impairment by the physical damage is an impairment by a cause selected from the group consisting of liver transplantation, cell transplantation and surgical partial hepatectomy.
[8] The hepatocyte replication promoter according to any one of [1] to [3] above wherein the impairment is an impairment caused by fatty hepatitis.
[9] The hepatocyte replication promoter according to any one of [1] to [8] above wherein the neutralizing agent for CXCL10 is a factor which is specifically bound to CXCL10 and inhibits an activity of CXCL10.
[10] The hepatocyte replication promoter according to [9] above wherein the factor which inhibits the activity is an anti-CXCL10 antibody.
[11] The hepatocyte replication promoter according to any one of [1] to [8] above wherein the neutralizing agent for CXCL10 is a factor which inhibits expression of CXCL10.
[12] The hepatocyte replication promoter according to any one of [1] to [8] above wherein the neutralizing agent for CXCL10 is an antagonist of a receptor for CXCL10.
[13] Use of a neutralizing agent for CXCL10 which is one of chemokines for producing a hepatocyte replication promoter.

[14] A method for promoting proliferation of hepatocytes comprising a step of administering to the hepatocytes an effective amount of a neutralizing agent for CXCL10 which is one of chemokines.
[15] The method for promoting the proliferation of the hepatocytes according to [14] above wherein the hepatocyte is a hepatocyte of a mammalian animal.
[16] The method for promoting the proliferation of the hepatocytes according to [15] above wherein the mammalian animal is a human being.
[17] The method for promoting the proliferation of the hepatocytes according to any one of [14] to [16] above wherein the neutralizing agent is administered to hepatic tissue having a disorder.
[18] The method for promoting the proliferation of the hepatocytes according to [17] above wherein the disorder is selected from the group consisting of an impairment caused by a toxic substance, an impairment by physical damage and an impairment caused by a pathogen.
[19] The method for promoting the proliferation of the hepatocytes according to [18] above wherein the impairment caused by the toxic substance is an impairment caused by a disease selected from the group consisting of alcohol induced hepatitis and drug induced hepatic disorders.
[20] The method for promoting the proliferation of the hepatocytes according to [18] above wherein the impairment by the physical damage is an impairment by a cause selected from the group consisting of liver transplantation, cell transplantation and surgical partial hepatectomy.
[21] The method for promoting the proliferation of the hepatocytes according to [18] above wherein the pathogen is hepatitis virus.
[22] The method for promoting the proliferation of the hepatocytes according to any one of [14] to [16] above wherein the disorder is an impairment caused by fatty hepatitis.
[23] The method for promoting the proliferation of the hepatocytes according to any one of [14] to [22] above wherein the neutralizing agent for CXCL10 is a factor which is specifically bound to CXCL10 and inhibits an activity of CXCL10.
[24] The method for promoting the proliferation of the hepatocytes according to [23] above wherein the factor which inhibits the activity is an anti-CXCL10 antibody.
[25] The method for promoting the proliferation of the hepatocytes according to any one of [14] to [22] above wherein the neutralizing agent for CXCL10 is a factor which inhibits expression of chemokine CXCL10.
[26] The method for promoting the proliferation of the hepatocytes according to any one of [14] to [22] above wherein the neutralizing agent for CXCL10 is an antagonist of a receptor for CXCL10.

[27] An insulin resistance ameliorating agent containing a neutralizing agent for CXCL10 which is one of chemokines as an active component. More specifically the insulin resistance ameliorating agent may comprise the neutralizing agent for CXCL10 which is one of the chemokines, and a pharmaceutically acceptable carrier.
[28] The insulin resistance ameliorating agent according to [27] above wherein insulin resistance in metabolic syndrome is ameliorated.
[29] The insulin resistance ameliorating agent according to [27] above wherein insulin resistance in type II diabetes is ameliorated.
[30] The insulin resistance ameliorating agent according to any one of [27] to [29] above wherein the neutralizing agent for CXCL10 is a factor which is specifically bound to CXCL10 and inhibits an activity of CXCL10.
[31] The insulin resistance ameliorating agent according to [30] above wherein the factor which inhibits the activity is an anti-CXCL10 antibody.
[32] The insulin resistance ameliorating agent according to any one of [27] to [29] above wherein the neutralizing agent for CXCL10 is a factor which inhibits expression of CXCL10.
[33] The insulin resistance ameliorating agent according to any one of [27] to [29] above wherein the neutralizing agent for CXCL10 is an antagonist of a receptor for CXCL10.
[34] Use of a neutralizing agent for CXCL10 which is one of chemokines for producing an insulin resistance ameliorating agent.

[35] A method for ameliorating insulin resistance comprising a step of administering to hepatocytes an effective amount of a neutralizing agent for CXCL10 which is one of chemokines.
[36] The method for ameliorating the insulin resistance according to [35] above wherein the hepatocyte is a hepatocyte of a mammalian animal.
[37] The method for ameliorating the insulin resistance according to [36] above wherein the mammalian animal is a human being.
[38] The method for ameliorating the insulin resistance according to [36] above wherein the mammalian animal is a patient exhibiting the insulin resistance.
[39] The method for ameliorating the insulin resistance according to [36] above wherein the mammalian animal is a patient with metabolic syndrome.
[40] The method for ameliorating the insulin resistance according to [36] above wherein the mammalian animal is a patient with type II diabetes.
[41] The method for ameliorating the insulin resistance according to any one of [35] to [40] above wherein the neutralizing agent for CXCL10 is a factor which is specifically bound to CXCL10 and inhibits an activity of CXCL10.
[42] The method for ameliorating the insulin resistance according to [41] above wherein the factor which inhibits the activity is an anti-CXCL10 antibody.
[43] The method for ameliorating the insulin resistance according to any one of [35] to [40] above wherein the neutralizing agent for CXCL10 is a factor which inhibits expression of chemokine CXCL10.
[44] The method for ameliorating the insulin resistance according to any one of [35] to [40] above wherein the neutralizing agent for CXCL10 is an antagonist of a receptor for CXCL10.

It has been reported that a plurality of chemokines and chemokine receptors are expressed in the hepatocyte upon disorder, and their involvement in the disorder has been speculated. It has been also known that CXCL10 is strongly expressed in the liver upon inflammation. However, it was completely unknown that the neutralizing agent for CXCL10 administered from an outside of the body directly contributes to the replication of mature hepatocytes, and its action to actually promote hepatic regeneration was discovered by the present inventors for the first time.

Furthermore, it will be described in detail in the following Examples 2 and 3 that the neutralizing agent for CXCL10 rapidly restores the construction of hepatic cords after injury to thereby keep the hepatic function damage to the mild level.

Therefore, according to the present invention, a new strategy is opened up for developing the therapy having a novel action tact *in vivo,* by which rapid replication of residual mature hepatocytes in the patient with severe hepatic disorder or dysfunction can be promoted to functionally ameliorate the liver while keeping the construction of the hepatic cords.

In addition to this, it is a finding which has been unknown in the prior art and has been revealed by the present inventors for the first time that CXCL10 administered from the outside of the body remarkably ameliorates the insulin resistance of the hepatocyte. Therefore, the present invention can become a basis of the therapy for the patients exhibiting the insulin resistance, e.g., the patients with type II diabetes or metabolic syndrome.

### EFFECT OF THE INVENTION

According to the present invention, the replication of the hepatocyte can be promoted. Therefore, according to the present invention, the impaired hepatic tissue can be regenerated.
Furthermore, according to the present invention, the insulin resistance can be ameliorated. Thus, the present invention is useful for the treatment of the diseases exhibiting the insulin resistance, e.g., type II diabetes and metabolic syndrome.

### BRIEF DESCRIPTION OF DRAWINGS

FIG. 1 is a graph showing change of expression of CXCL10 mRNA in CCl₄-induce hepatic disorder.
FIG. 2 is a view showing immunologically stained images of CCl₄-induced hepatic disorder.
FIG. 3 is a graph showing the change of weight of livers with CCl₄-induced hepatic disorder.
FIG. 4 is a graph showing the change of serum ALT levels in CCl₄-induced hepatic disorder.
FIG. 5 is a graph showing the change of necrotic areas in CCl₄-induced hepatic disorder.
FIG. 6 is a view showing images of CD31-staining positive cells in a sinusoid region with CCl₄-induce hepatic disorder (3 days after administering CCl₄).
FIG. 7 is a graph showing quantitative data of BrdU positive proliferating hepatocytes in the sinusoid region with CCl₄-induced hepatic disorder (3 days after administering CCl₄).
FIG. 8 is a combination of graphs showing effects of anti-CXCL10 antibody on hepatic tissue stem cells with CCl₄-induced hepatic disorder. An upper panel, a middle panel and a lower panel show, respectively, test results on the day 8 weeks after birth (8w), control results 3 days (d3) after administrating CCl₄ and test results of administrating the anti-CXCL10 antibody 3 days after administrating CCl₄.
FIG. 9 is a graph showing the effect of anti-CXCL10 antibody administration on normal mice.
FIG. 10 is a graph showing the effect of a recombinant CXCL10 protein on normal mice.
FIG. 11 is a view showing an electrophoretic profile which detected the expression of CXCL10 receptor in human hepatic cell line HepG2.
FIG. 12 is a graph showing the effect of the anti-CXCL10 antibody on proliferation of HepG2.
FIG. 13 is a graph showing amelioration of abnormal glucose tolerance by the anti-CXCL10 antibody.
FIG. 14 is a graph showing the amelioration of insulin resistance by the anti-CXCL10 antibody.

### EXPLANATIONS OF LETTERS OR NUMERALS

P Portal vein portion
F Portion stained with fluorescence
CD31 CD31 positive portion
Nuclei Stained nucleus portion
ND Not done

### BEST MODES FOR CARRYING OUT THE INVENTION

The present invention is described for limited numbers of embodiments, but it is to be understood that many variations, modifications and other adaptations of the present invention can be performed.

The hepatocyte replication promoter of the present invention is characterized by containing a neutralizing agent for CXCL10 which is one of chemokines as an active component. The hepatocyte replication promoter of the present invention may be composed as a formulation containing the neutralizing agent for CXCL10 and a pharmaceutically acceptable carrier. The method for promoting hepatocyte proliferation of the present invention comprises a step of administering an effective amount of the neutralizing agent for CXCL10 to the hepatocyte.

As one preferable embodiment of the present invention, the replication of the hepatocyte in the impaired hepatic tissue is promoted. The hepatocyte replication promoter of the present invention is useful when proliferation of the hepatocyte is intended regardless of the cause of the impairments. For example, the hepatocyte replication promoter of the present invention is suitably applied to a hepatic tissue having an impairment caused by a toxic substance, the impairment by physical damage or the impairment caused by a pathogen. Examples of the impairment caused by the toxic substance may include impairments due to diseases such as alcohol induced hepatitis and drug induced hepatic disorders. As examples of the pathogen, hepatitis virus is exemplified. As examples of the impairment by the physical damage, the impairments caused by liver transplantation, cell transplantation and surgical partial hepatectomy are exemplified. Furthermore, the hepatocyte replication promoter of the present invention can be applied to fatty hepatitis. Among fatty hepatitis, in particular, non-alcohol-dependent hepatitis is one of lifestyle related diseases whose relevance with metabolic syndrome is pointed out, and the present invention is also useful for the treatment of this non-alcohol-dependent hepatitis.

In one preferable embodiment of the present invention, a therapeutically effective amount of the neutralizing agent for CXCL10 is administered to a subject who lost mature hepatocytes or has a functional disorder of mature hepatocytes associated with impaired hepatic tissue or has a possibility of causing such a loss or disorder, to' thereby decrease or restore severe tissue disorder and functional disorder in the subject. The present invention is clinically useful for regeneration or restoration of the hepatic tissue having acute hepatic failure, chronic hepatic failure or hepatic disorders caused by nervous diseases. The subject may be mammals including human beings.

As used herein, the "neutralizing agent for CXCL10" refers to a medicament having an action to inhibit an activity of physiological functions of CXCL10. The physiological functions of CXCL10 mean the functions exerted by CXCL10, and specifically may include the functions thereof exerted by binding to CXR3. In one preferable embodiment of the present invention, the neutralizing agent is a factor which inhibits the activity of CXCL10 and at least promotes the replication of the mature hepatocyte. As the neutralizing agent, a factor which specifically binds to CXCL10 for inhibiting the activity of CXCL10 and a factor inhibiting the expression of CXCL10 are also exemplified.

As one embodiment, the neutralizing agent may be any molecule which binds to CXCL10 with sufficient affinity to decrease the CXCL10 activity. Examples of the neutralizing agent may preferably include a binding molecule such as an antibody against CXCL10 (anti-CXCL10 antibody). Furthermore, fragments and analogue peptides of the CXCR3 receptor which bind to CXCL10 with sufficient affinity to decrease the CXCL10 activity are also exemplified. In addition, enzymes which specifically decompose CXCL10 are also exemplified. Examples of the neutralizing agent for CXCL10 may include not only substances which directly act upon CXCL10 to inhibit the activity but also substances which act upon a substance other than CXCL10 to inhibit the CXCL10 activity. Examples of such a neutralizing agent may include antagonists of the receptor for CXCL10. The receptor for CXCL10 may include receptors present on the hepatocyte. For example, CXCR3 such as CXCR3-A and CXCR3-B may be included.

The anti-CXCL10 antibody may be prepared as a polyclonal antibody or a monoclonal antibody.
Methods for eliciting the polyclonal antibody in rabbits, goats, mice or other mammalian animals are well-known in the art. Production of an anti-peptide antibody typically comprises the use of a host animal such as rabbits, mice, guinea pigs or rats. When the serum in a large amount is needed, a large animal such as a sheep, goat, horse, swine or ass may be used. The animal is typically selected depending on the needed amount of the antiserum. The suitable animals may include rabbits, mice, rats, guinea pigs and hamsters. The amount of serum obtained from these animals per one blood drawing is typically maximum 25 mL from a rabbit, 100 to 200 µL from a mouse, and 1 to 2 mL from other animals. For example, 15 to 50 µg of an antigen in an appropriate adjuvant such as Freund's complete adjuvant may be injected twice with an interval of 2 to 4 weeks, subsequently the blood may be collected and an antiserum may be analyzed.

The monoclonal antibody may be obtained using the methods well known and used commonly in the art. A peptide portion of the protein (e.g., CXCL10) used as an immunogen may be determined using the method well-known in the art. Spleen cells derived from the mouse immunized with CXCL10 may be fused with appropriate myeloma cell line to generate hybridoma cells. Cloned hybridoma cell lines may be screened using a labeled CXCL10 protein in order to identify the clone secreting the anti-CXCL10 antibody. A hybridoma which has the desired specificity and affinity and expresses the anti-CXCL10 monoclonal antibody may be isolated, and utilized as a sustained supply source of the antibody neutralizing agent.

A recombinant antibody, e.g., a chimera antibody and a humanized antibody may be obtained by the methods known in this field. The humanized antibody may be constructed by imparting an essentially arbitrary antigen binding specificity to a human antibody framework. The method for constructing the humanized antibody is useful for carrying out the method of the present invention, and useful for preparing the suitable antibody neutralizing agent in order to avoid an immune response in the host to the antibody neutralizing agent when therapeutically used.

Furthermore, the neutralizing agent may be a regulatory molecule which inhibits or promotes the function of a CXCL10 regulatory protein or the gene region thereof and decreases the degree, the amount or the speed of CXCL10 expression or the activity thereof, or any molecule which binds to such a gene region. Examples of the neutralizing agent which decreases the CXCL10 expression may include antisense nucleic acids and transcription inhibitors.

In one embodiment of the present invention, a polynucleotide encoding CXCL10, a regulatory molecule which regulates the expression or the activity of CXCL10, and any fragments or antisense molecules thereof may be used as the neutralizing agent. In one aspect, transcription or translation of CXCL10 mRNA may be blocked using the antisense molecule for the nucleic acids encoding CXCL10. Particularly, cells may be transformed with a sequence complementary to the nucleic acids encoding CXCL10. Such methods are well-known in the art. Sense or antisense oligonucleotides or larger fragments may be designed on the basis of various positions in a coding region or a control region of the sequence encoding CXCL10. Using the antisense molecule obtained in this manner, it is possible to neutralize the CXCL10 activity or accomplish the regulation of the gene function. In the present invention, a ribozyme, an RNAi active molecule (siRNA) and the like which are specific for CXCL10 may also be used as the neutralizing agent.

The method for promoting the hepatocyte proliferation of the present invention is applicable to all cases of intending to proliferate the hepatocyte regardless of its purpose such as therapeutic purposes and academic purposes. Preferable embodiments of the method for promoting the hepatocyte proliferation of the present invention may include a treatment of the subject having the disorder in the hepatocytes, and a regeneration of hepatocytes in a donor of the hepatic tissue in the hepatic tissue transplantation or a patient who has undergone the hepatic tissue transplantation.

In the present invention, when the neutralizing agent is administered, conditions such as an effective amount and formulation may be appropriately set up depending on the type and the purpose of the neutralizing agent. For example, the neutralizing agent useful for carrying out the method of the present invention may be formulated and administered by those skilled in the art in a suitable manner and amount in the manner which adapts to severity of hepatocyte injury to be treated; a speed and quantity of the injury; body weight, gender, age and health state of the subject; biochemical natures, biological activity, viability and side effects of a particular compound; and a procedure regimen to be concurrently used. The suitable amount and formulation may be presumed from a reliable animal model for the particular disorder known publicly in the art. It is understood that a dosage of the neutralizing agent for CXCL10 must be adjusted based on the binding affinity of the neutralizing agent for CXCL10 so that the neutralizing agent exhibiting significantly and higher binding affinity is administered in a lower dose compared with the dosage required for the neutralizing agent having the lower binding affinity. Therefore, the suitable dosage varies depending on the particular procedure and a desired period of time for the procedure. Typically, the suitable dosage for therapeutic procedure may be appropriately determined in the range between about 10 µg to about 1 mg per kg body weight per day. The dosage when administered as a physiologically acceptable composition may be determined in a range by which a plasma concentration of generally 0.1 µg/mL or highrer, preferably 1.0 µg/mL or higher and more preferably about 2 µg/mL or higher is sufficiently accomplished. Specifically, it is desirable to determine the dose in the range to sufficiently accomplish the plasma concentration of about 0.1 µg/mL to about 100 µg/mL, preferably about 1.0 µg/mL to about 50 µg/mL, more preferably about 2 µg/mL to about 50 µg/mL and particularly preferably 5 to 10 µg/mL.

The neutralizing agent which is the active component of the hepatocyte replication promoter of the present invention may be administered to the subject through a variety of routes known publicly in the art (e.g., systemic administration (e.g., intravenous administration)). The neutralizing agent for CXCL10 may be provided in a form of an isolated and substantially purified polypeptide and polypeptide fragment in a pharmaceutically acceptable formulation using a formulation method publicly known to those skilled in the art. These formulations may be administered through standard routes (topical route, percutaneous route, intraperitoneal route, oral route, rectal route) or parenteral routs (e.g., intravenous route, subcutaneous route or intraportal vein route). The intravenous administration of the neutralizing agent for CXCL10 is the particularly suitable route for carrying out the method of the present invention. The route through hepatic artery may be used for targeting the delivery of the neutralizing agent to the impaired portion or the injured portion. Furthermore, a modified neutralizing agent for CXCL10 may be incorporated into a biodegradable polymer, and this enables to slowly release the compound useful for reducing the hepatic disorder. For the hepatocyte replication promoter of the present invention, a suitable dosage form may be selected based on the conditions such as administration route and administration method as described above.

The neutralizing agent for CXCL10 may be administered together with a pharmaceutically acceptable medium to be in a form of a solution or a suspension. For example, such a pharmaceutically acceptable medium may be, for example, sterile water-based solvents (e.g., sodium phosphate buffer, phosphate buffered saline, normal saline or Ringer solution, or other physiologically buffered saline), or other solvent or vehicles (e.g., glycol, glycerol, oils (e.g., olive oil), or injectable organic ester). The pharmaceutically acceptable medium may further comprise physiologically acceptable compounds (e.g., compounds which stabilize the neutralizing agent, compounds which increase its solubility or compounds which increase its absorbance). Examples of such a physiologically acceptable compound may include sugars (e.g., glucose, sucrose or dextran); antioxidants (e.g., ascorbic acid or glutathion); chelating agents (e.g., EDTA) (this destroys microbial membranes); bivalent metal ions (e.g., calcium or magnesium); low molecular weight proteins; lipids or liposomes; or other stabilizers and excipients. Those skilled in the art appreciate that the selection of the pharmaceutically acceptable carrier depends on the administration route of the compounds including the neutralizing agent, and particular physical and chemical characteristics thereof.

Examples of the formulations suitable for the parenteral administration may include water-based and non-water-based sterile solutions for injection (e.g., the aforementioned pharmaceutically acceptable medium). The solution may further comprise, for example, a buffer, a bacteriostatic agent, and a solute which makes the solution isotonic with the blood of an intended recipient receiving the formulation. Examples of other formulations may include water-based and non-water-based sterile suspensions which may contain a suspending agent and a thickening agent. The formulation may be presented in a unit dose container or a multiple dose container (e.g., sealed ampoule and sealed vial), and may be stored in a lyophilized state which requires the addition of a sterile liquid carrier just before the use. An immediate injectable solution and an immediate injectable suspension may be prepared from sterile powders, granules and tablets of the types previously described.

According to another embodiment of the present invention, there is a treatment system comprising the neutralizing agent for CXCL10 and instructions for the use in the method for decreasing the severity of the hepatocyte injury, which is preferably presented in a kit form. In one embodiment, for example, the therapeutic agent is the anti-CXCL10 antibody.

A suitable kit comprises at least one CXCL10 neutralizing agent (neutralizing agent for CXCL10) as a separately packed chemical reagent in a sufficient amount for at least one treatment application. Instructions for the use of the packed kit may also be contained. Those skilled in the art may easily incorporate the CXCL10 neutralizing agent with the suitable buffer and solution for carrying out the method of the present invention as described herein to be in a form of a kit.

As described above, the present inventors have confirmed that, simultaneously with promoting the replication of the hepatocyte, the neutralizing agent for CXCL10 also exhibit the action of increasing the reactivity and sensitivity to insulin and ameliorate the abnormal glucose tolerance, and that the neutralizing agent for CXCL10 is thus useful as the active component of the insulin resistance ameliorating agent.
The present invention provides as another embodiment the insulin resistance ameliorating agent containing the neutralizing agent for CXCL10 as the active component.

In the present invention, the amelioration of the insulin resistance specifically means augmenting the reactivity and sensitivity of the hepatocyte in the liver to insulin. In particular, the amelioration includes alteration of the reactivity and sensitivity of the hepatocyte in an individual exhibiting the insulin resistance to the same as or close level to those of the normal individual. It is speculated that amelioration of the insulin resistance by the neutralizing agent for CXCL10 proceeds via the following action mechanism. First, when the neutralizing agent for CXCL10, e.g., the anti-CXCL10 antibody is administered, at least the binding of the CXCL10 to its receptor is inhibited on the hepatocytes (mainly mature hepatocytes) and simultaneously, β-catenin which is a protein in a hepatocyte membrane migrates into a nucleus in the cell. Subsequently, the expression of a protein c-Myc is activated. As a result, the amount of insulin bound to the insulin receptor on the hepatocytes is increased. That is, the reaction of the liver to insulin is enhanced and the amelioration of sugar metabolism in the liver is promoted. The action mechanism for promoting the replication of the hepatocytes is also the action of the neutralizing agent for CXCL10 upon the hepatocytes. Thus, it is speculated that this action mechanism and the action mechanism for the amelioration of the insulin resistance are entirely or at least partially (e.g., until the control of β-catenin signal) common.

The insulin resistance ameliorating agent of the present invention ameliorates the insulin resistance as a result of action of the neutralizing agent for CXCL10 upon the hepatocytes as described above. Therefore, in the same way as the hepatocyte replication promoter, the insulin resistance ameliorating agent targets the hepatocyte. Thus, the amount of the neutralizing agent for CXCL10 to be used and the administration condition may be the same as those in the aforementioned hepatocyte replication promoter.

As one preferable embodiment of the present invention, the insulin resistance in the hepatocytes having the insulin resistance, e.g., the hepatocytes derived from the mammalian animals including human beings is ameliorated. The hepatocytes may be those extracted from the individual exhibiting the insulin resistance and then purified and cultured. In another preferable embodiment of the present invention, the insulin resistance in a subject is ameliorated by administering the therapeutically effective amount of the neutralizing agent for CXCL10 to the subject exhibiting the insulin resistance. The present invention is useful for ameliorating the abnormal glucose tolerance in patients exhibiting the insulin resistance, e.g., patients with type II diabetes (NIDDM) or metabolic syndrome. Although it is not scientifically elucidated how the insulin resistance and abnormal sugar metabolism (abnormal glucose tolerance) are positioned in the mechanism of the metabolic syndrome, it is obvious that these are involved in the mechanism. The prevalence rate of the metabolic syndrome is considered to be fairly high. Thus, the present invention is useful as those ameliorating its symptoms and contributing to the treatment.

### EXAMPLES

The present invention is further exemplified by the following non-limiting Examples. The following Examples intend to exemplify the present invention, but do not intend to limit the present invention. It is understood that modifications which do not substantially affect the activity on a variety of embodiments of the present invention are included within the definition of the present invention provided herein. Therefore, the following Examples intend to exemplify the present invention, but do not intend to limit the present invention.

### (Example 1) Augmentation of CXCL10 expression in impaired hepatocytes using carbon tetrachloride induced hepatic disorder model

The expression of CXCL10 was evaluated using the carbon tetrachloride induced hepatic disorder model. The carbon tetrachloride induced hepatic disorder model was constructed in accordance with the description in Morrison GR. Arch. Biochem. Biophys. 111:448, 1965. Because of no infiltration of Th1 cells in this hepatic disorder model, the effect of CXCL10 on Th1 cells can be ruled out. Thus, this model has an advantage that replication and regeneration of hepatocytes *in vivo* may be purely evaluated. The nucleotide sequence of CXCL10 has been already registered in database such as DDBJ (DNA Data Bank of Japan). The CXCL10 protein and the anti-CXCL10 antibody are commercially available products.

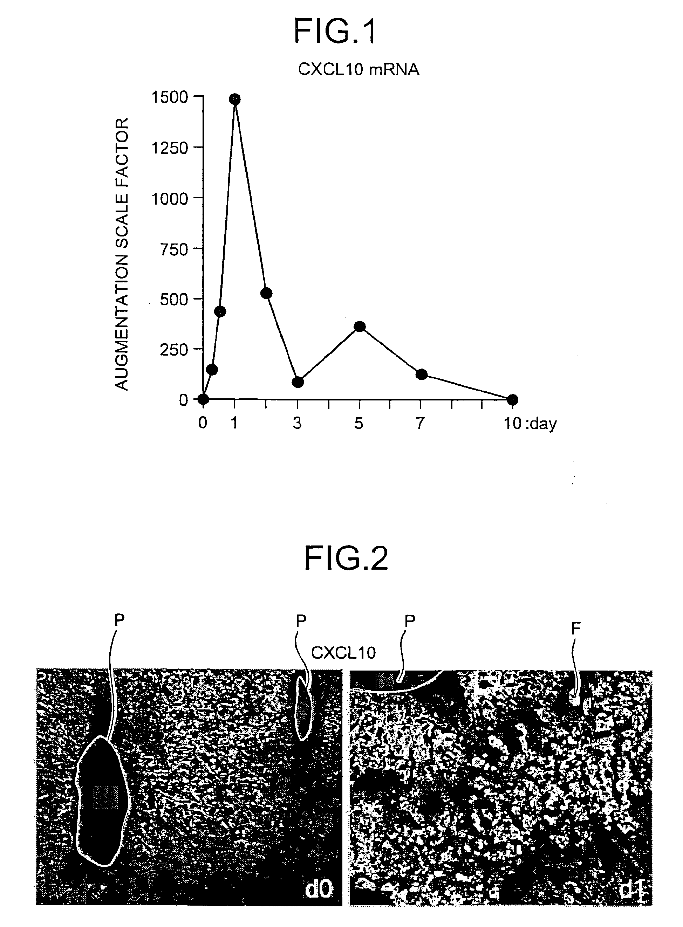

Carbon tetrachloride (CCl₄ 0.5 µL/g body weight supplied from Sigma Aldrich) was intraperitoneally injected into C57BL6/J mice (female, 8 to 9 weeks of age supplied from CLEA Japan Inc.). The mice were sacrificed before CCl₄ administration and 1, 6, 12 hours, and 1, 2, 3, 5, 7 and 10 days after the administration, and the liver was collected. A part of the first lobe of the liver was immediately frozen in a liquid nitrogen, and total RNA was extracted using an RNAzol reagent (supplied from BIOTECX LAB) and reversely transcribed to yield cDNA. The expression of CXCL10 was analyzed by real-time quantitative PCR with 40 amplification cycles at 95°C for 15 seconds and 55°C for one minute and 30 seconds using ABI7700 sequence detector system (supplied from PE Applied Biosystems). The expression amount of CXCL10 in each sample was calibrated with the expression amount of GAPDH which was an endogenous control. Subsequently the relative expression amount was calculated based on the expression amount of CXCL10 before the administration of CCl₄, and this was rendered an augmentation scale factor. The change of the augmentation scale factor with time is shown in FIG. 1.

A part of the second lobe of the liver was embedded in an OTC compound (supplied from Miles) and a frozen block thereof was prepared in the liquid nitrogen. Sections of 6 µm were prepared using a cryostat (supplied from Microm), and reacted using the anti-CXCL10 antibody (goat polyclonal antibody, 20 µg/mL, supplied from Santa Cruz) or an anti-CXCR3 antibody (goat polyclonal antibody, 20 µg/mL, supplied from Santa Cruz) at room temperature for one hour. Subsequently, the sections were further reacted with Alexa-594 labeled anti-goat IgG (supplied from Molecular Probe, diluted at 1:200) at room temperature for 30 minutes. The sections was mounted with an anti-fade mounting medium (supplied from DAKO), then observed under a confocal fluorescence laser microscope (TCS SP2 AOBS; Leica Microsystems), and photographed.

The results of the quantitative PCR (change of augmentation scale factor with time) are shown in FIG. 1. CXCL10 mRNA was constitutively expressed in the liver, and its expression was dramatically augmented by the administration of CCl₄. The expression of CXCL10 mRNA was augmented one hour after the administration of CCl₄. The first maximum peak of the expression was exhibited one day after the administration, and the second peak was shown 5 days after the administration, i.e., a bimodal manner of the expression augmentation was exhibited.

Immunologically stained pictures (200 times) are shown in FIG. 2. In FIG. 2, the left picture shows a staining pattern of the tissue before the administration of CCl₄, and the right picture shows a staining pattern of the tissue after the administration of CCl₄ (one day thereafter). In FIG.2, P indicates a portal vein portion, and F indicates a stained portion. As shown in FIG. 2, the CXCL10 protein was also constitutively detectable in the hepatocytes in the immunostaining. CXCL10-positive hepatocytes were remarkably increased 6 hours after the administration of CCl₄, and their number reached the peak one day after the administration. The dramatic augmentation of CXCL10 expression in the hepatic disorder was identified at both an mRNA level and a protein level. It was also shown that the expression was localized in the impaired hepatocytes.

### (Example 2)

For the purpose of analyzing whether the augmentation of CXCL10 expression is beneficial or harmful for the living body, 100 µg of a monoclonal anti-CXCL10 antibody (suspended in 200 µL of sterile PBS) was once injected into a tail vein of C57BL6/J mice (female, 8 weeks of age, supplied from CLEA Japan Inc.) just before administering CCl₄. The monoclonal antibody used in this study is specific for CXCL10 and exhibits no immunological cross reactivity with CXCL9 and CXCL11 which are other ligands of its receptor CXCR3. As its neutralization activity, the monoclonal antibody inhibits chemotaxis of CXCR3 positive activated T lymphocytes. The mice were sacrificed with time, and clinical findings were compared with those in the mice treated with a control antibody.

The monoclonal antibody was prepared using methods well-known in the art and used commonly. A peptide portion of the CXCL10 protein for use as an immunogen was determined by the method well-known in the art, and a recombinant CXCL10 protein was prepared (such a recombinant protein is easily available from R & D). Spleen cells derived from a mouse immunized with CXCL10 were fused with myeloid cells to produce hybridoma cells. Cloned hybridoma cells were screened using the biotin-labeled CXCL10 protein to identify clones secreting the anti-CXCL10 antibody. The hybridoma expressing the anti-CXCL10 monoclonal antibody having the specificity and the affinity can be isolated and utilized as a sustained supply source.

As to the antibody thus prepared, the neutralization activity and the specificity were confirmed as follows. CHO-K1 cells were transfected with murine CXCR3 (receptor for CXCL10) by the method well-known in the art to prepare a CHO-K1 cell line which constitutively expressed CXCR3. In an experiment system in which the murine recombinant protein is added to the culture of this cell line and its effect is evaluated by calcium influx assay, it was confirmed that calcium reaction was inhibited by adding the monoclonal anti-CXCL10 antibody. It was also confirmed by immunoprecipitation test that the monoclonal anti-CXCL10 antibody exhibited no cross reactivity with CXCL9 and CXCL10 which were other ligands of CXCR3.

As the control antibody, a monoclonal antibody against a human parathyroid-related peptide belonging to the subclass IgGl which is the same as the anti-CXCL10 antibody was used. This antibody is available from several companies such as Santa Cruz and Chemi-Con. In the following Examples, this monoclonal antibody was used as the control antibody as well.

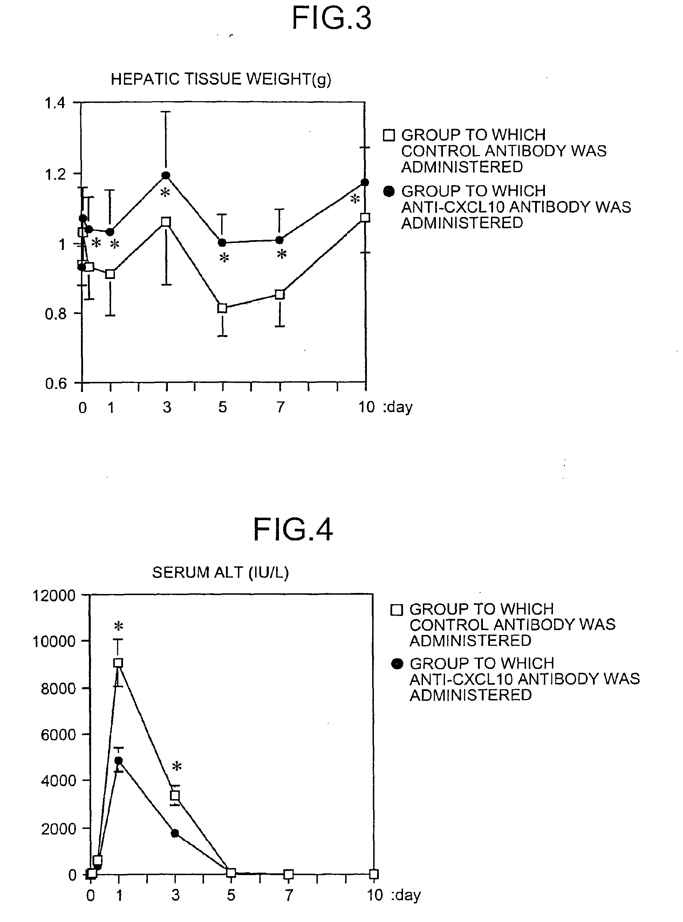

The change of murine liver weight (weight of liver tissue) is shown in FIG. 3. In FIG. 3, "*" indicates that a significant difference at p<0.05 by t-test was observed. In the group treated with the control antibody, the liver weight was rapidly decreased until one day after administering CCl₄, and gradually recovered within 3 days. Meanwhile, in the group treated with the anti-CXCL10 antibody, the liver weight was also decreased one day after administering CCl₄ but was rapidly recovered compared with the group treated with the control antibody. This indicates that the regeneration of the impaired liver is rapidly promoted by treating with anti-CXCL10 antibody.

The change of murine serum ALT (alanine transferase) levels (IU/L) is shown in FIG. 4. In FIG. 4, "*" indicates that a significant difference at p<0.05 by t-test was observed. Serum ALT is the most reliable clinical indicator for observing the degree of the impaired hepatocytes, and was measured using Fuji Drychem slide (supplied from Fuji Medical Systems Co., Ltd.). In the group treated with the control antibody, the ALT level was dramatically elevated after administering CCl₄, reached the peak one day thereafter, gradually decreased from the 3rd day and recovered to the normal range on the 7th day. Meanwhile, in the group treated with the anti-CXCL10 antibody, the elevation of the ALT values was significantly suppressed, and the levels were recovered to the normal range on the 5th day. It was found that injury of the hepatocytes was dramatically suppressed by treating with the anti-CXCL10 antibody.

### (Example 3)

Just before administering CCl₄, 100 µg of the monoclonal anti-CXCL10 antibody (suspended in 200 µL of sterile PBS) or the control antibody was once injected into the tail vein of C57BL6/J mice (female, 8 weeks of age, supplied from CLEA Japan Inc.). The mice were sacrificed with time, and histological pathological findings were compared with those in the group treated with the control antibody. Frozen sections of the liver were prepared by the method described in Example 1, and analyzed immunohistologically. As a primary antibody, a rat anti-murine CD31 monoclonal antibody (2 µg/mL, supplied from PharMingen) or a rabbit anti-murine collagen type IV polyclonal antibody (diluted at 1:250, supplied from Sigma) was reacted at room temperature for one hour. Subsequently, alkaline phosphatase-labeled anti rat or rabbit IgG (diluted at 1:200, supplied from Jackson ImmunoResearch) was reacted at room temperature for one hour. The binding of the antibody was visualized under an optical microscope (supplied from Leica) using a coloring substrate for alkaline phosphatase (Vector Red, supplied from Vector Laboratory).

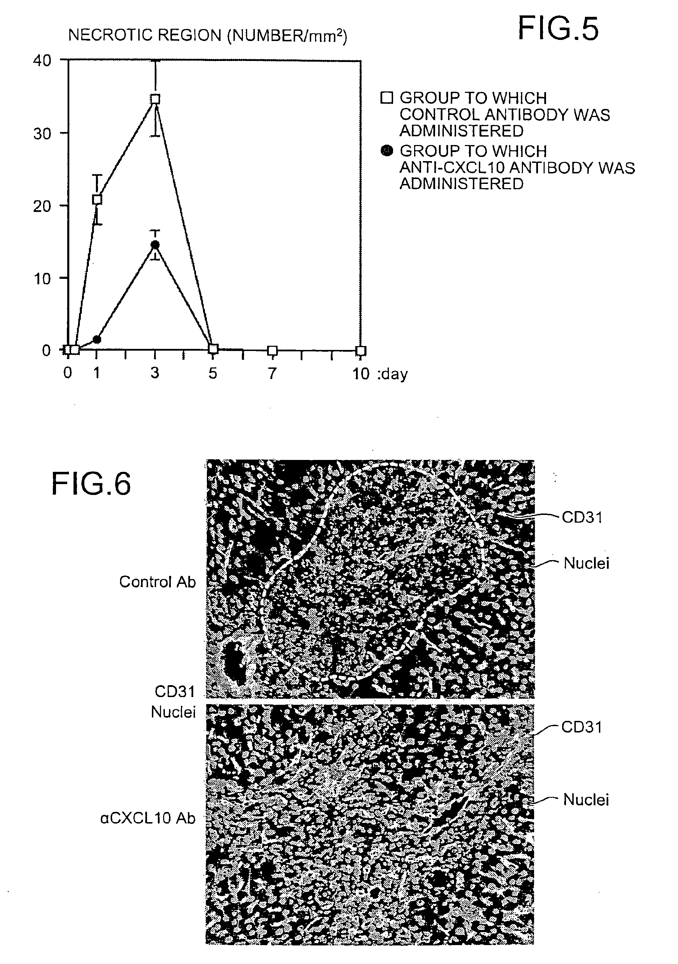

The change of necrotic areas in the hepatic tissue is shown in FIG. 5. The substantial hepatocytes may be specified by staining collagen type IV. By staining collagen type IV, the construction of hepatic cords was defined to determine a region of cell necrosis, and the area of the region was calculated using Win Roof image software. The region of at least 15 mm² was measured on the liver section. In the group treated with the control antibody, multiple widespread hepatocyte necrosis mainly around the central venous region was observed. On the contrary, in the group treated with the anti-CXCL10 antibody, the necrotic region was dramatically diminished.

The anti-CD31 antibody is the monoclonal antibody which reacts with vascular endothelial cells, and is used for detecting sinusoid endothelial cells in the liver. Images (magnification 20 times) obtained by immunologically staining with the anti-CD31 antibody on 3 days after administering CCl₄ are shown in FIG. 6. In the group to which the control antibody was administered (upper image in FIG. 6), the construction of sinusoid walls was broken down due to the necrosis as shown in the region surrounded with a dashed line. On the contrary, in the group treated with the anti-CXCL10 antibody (lower image in FIG. 6), the sinusoid wall was clearly constructed. These results indicate that the anti-CXCL10 antibody inhibits the hepatocyte injury pathologically and regenerates the construction of the sinusoid wall rapidly.

### (Example 4)

In order to examine whether the regeneration of hepatocytes actually occurs in *vivo* or not, uptake of BrdU (5-bromo-2'-deoxyuridine/deoxyuridine bromide) which is the indicator of proliferating cells was analyzed. BrdU is a thymidine analogue and is incorporated in DNA during an S phase of a cell cycle. In order to label the tissue with BrdU, BrdU (supplied from Sigma, 500 µg of BrdU suspended in 200 µL of sterile PBS) was injected into the tail vein of the mouse treated with the anti-CXCL10 antibody or the control antibody after eliciting the hepatic disorder by CCl₄ in the same way as in Examples 2 and 3. One hour thereafter, the mouse was sacrificed, the second lobe of the liver was collected and the frozen sections were prepared by the method in Example 1. Collagen type IV was visualized by the method in Example 3, and subsequently an immunostaining (BrdU staining kit supplied from Zymed) using an anti-BrdU antibody was given for specifically detecting nuclei in the S phase. The quantification on the tissue was performed as described in Example 3.

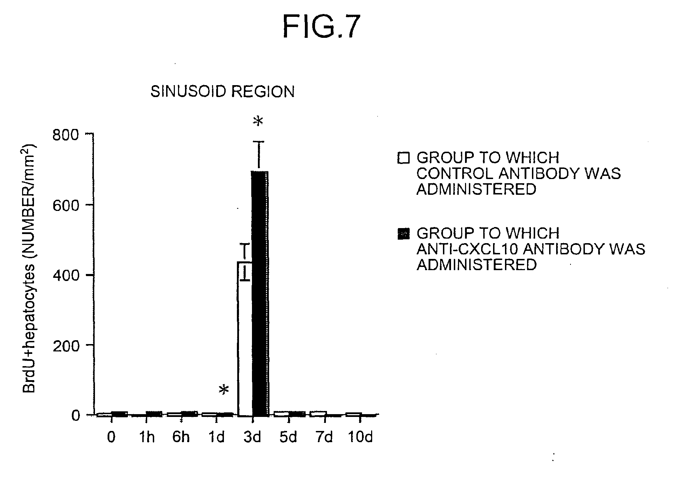

Data of quantitative analysis are shown in FIG. 7. In FIG. 7, "*" indicates that a significant difference at p<0.05 by t-test was observed. BrdU-positive cells are observed in not only hepatic parenchymal cells but also non-parenchymal cells such as immune cells. Thus, only the hepatocytes were correctly counted by double staining with e.g., collagen type IV. A vertical axis (BrdU + hepatocytes [number/mm²]) in FIG. 7 represents the number of cells doubly stained with the anti-BrdU and the anti-collagen type IV antibodies per unit area. Compared with the group treated with the control antibody, in the group treated with the anti-CXCL10 antibody, the number of the BrdU-positive hepatocytes was remarkably increased. The BrdU-positive cells were quantified in the sinusoid region. Consequently, in the sinusoid region in the group treated with the anti-CXCL10 antibody, the number of the BrdU-positive cells was increased from the CCl₄ administration until 3 days after the administration, but the number of the BrdU-positive cells was conversely decreased on the 7th day to the 10th day. This result indicates that the hepatocytes, especially the mature hepatocytes in the sinusoid region rapidly proliferate by treating with the anti-CXCL10 antibody and that the hepatocytes return to a resting phase promptly after the regeneration of hepatic cords. That is, it has been demonstrated *in vivo* that the anti-CXCL10 antibody may promote the hepatic regeneration after the injury.

### (Example 5) Effect on hepatic tissue stem cells

When BrdU (50 µg of BrdU suspended in 10 µL of sterile PBS) is subcutaneously injected into neonatal mice twice daily for 3 days from the 3rd day to the 5th day after the birth and the course is followed up for 8 weeks, hepatocytes having a slow cell cycle and leaving a BrdU label for a long time are detected. Such a cell leaving the BrdU label for a long time is referred to as a label retaining cell (LRC), and it has been known that LRC is in the resting phase in the mouse aged 8 weeks and has the nature as the stem cell (Review, Fuchs E, et al. Cell 116:769, 2004). In the liver, LRC is occasionally observed in a very small number (about 7% in total hepatocytes) in the portal vein region, and is thought to be a hepatic tissue stem cell which is different from the mature hepatocyte. The CCl₄-induced hepatic disorder was elicited in the mice (8 weeks of age) given such a label followed by administering the anti-CXCL10 antibody or the control antibody in the same way as in Examples 2 to 4. The mice were sacrificed 3 days thereafter, the second lobe of the liver was collected and the frozen sections were prepared. The hepatocytes which had been proliferating upon sacrificing and LRC were detected simultaneously by double immunostaining using an anti-cyclin A antibody (100 µg/mL, goat polyclonal antibody, supplied from Santa Cruz) which specifically detected the nuclei in the S phase in the cell cycle and anti-BrdU antibody. As the second antibody, Alexa-488 labeled anti-goat IgG (diluted at 1:200, supplied from Molecular Probe) and Alexa-594 labeled streptoavidin were used. The quantification on the tissue was performed as described in Example 3.

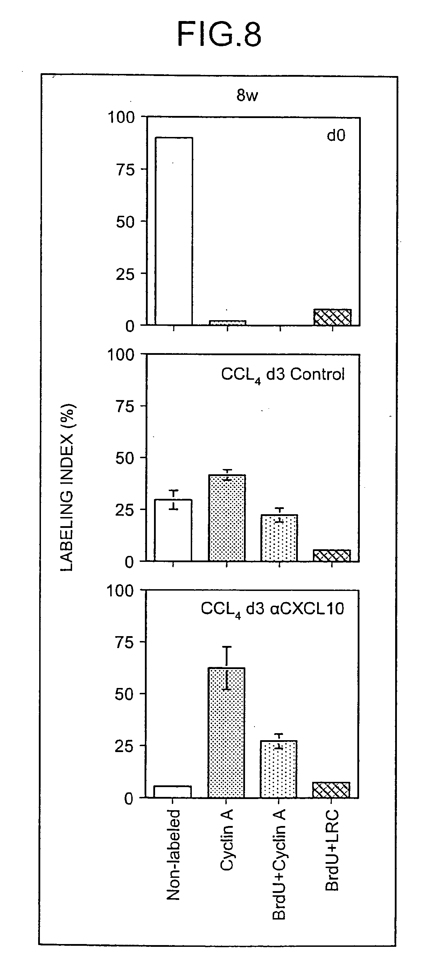

Data of quantitative analysis are shown in FIG. 8. In FIG. 8, the upper, middle and lower panels show, respectively, the result before the administration, the result in the group treated with the control antibody and the result in the group treated with the anti-CXCL10 antibody. In FIG. 8, a "labeling index (%)" represents an area percentage of each labeled portion relative to 100% of total hepatocytes displayed in the image. In the group treated with the control antibody (middle panel in FIG. 8), the proliferating hepatocytes (cyclin A positive cells) in the liver on 3 days after the CCl₄ administration was 64.5% (mean; n=5, 15 mm² section, which are the same as those in other experiments) in the total hepatocytes. In this, the proliferating cells derived from LRC (cyclin A positive and BrdU positive) was 34.5% (22.3% in the total hepatocytes) and the proliferating cells thought to be derived from the mature hepatocytes (cyclin A positive and BrdU negative) was 65.6% (42.3% in the total hepatocytes). Actually, binuclear mature hepatocytes immediately after cell division were also observed. That is, it has been revealed that the rapid cell division and replication of not only the hepatic tissue hepatic stem cells (LRC) but also the mature hepatocytes are involved in the regeneration of hepatocytes after the hepatic disorder. On the contrary, in the group treated with the anti-CXCL10 antibody (lower panel in FIG. 8), the proliferating cells was observed to be 88.4%. Contents thereof were 30% of the cells derived from LRC (26.5% in the total hepatocytes) and 70% of the cells derived from the mature hepatocytes (61.8% in the total hepatocytes). This indicates that the anti-CXCL10 antibody significantly promoted the replication of the mature hepatocytes.

### (Example 6) Effects of anti-CXCL10 antibody on liver in normal mouse

The anti-CXCL10 antibody or the control antibody was injected into the tail vein of C57BL6/J mice (female, 8 weeks of age, supplied from CLEA Japan Inc.) in the normal state. The mice were sacrificed 6 hours thereafter, the second lobe of the liver was collected and the frozen sections were prepared. In the same way as in Example 4, BrdU (500 µg of BrdU suspended in 100 µL of sterile PBS) was injected into the tail vein one hour before sacrificing, and the immunostaining with anti-BrdU antibody was performed.

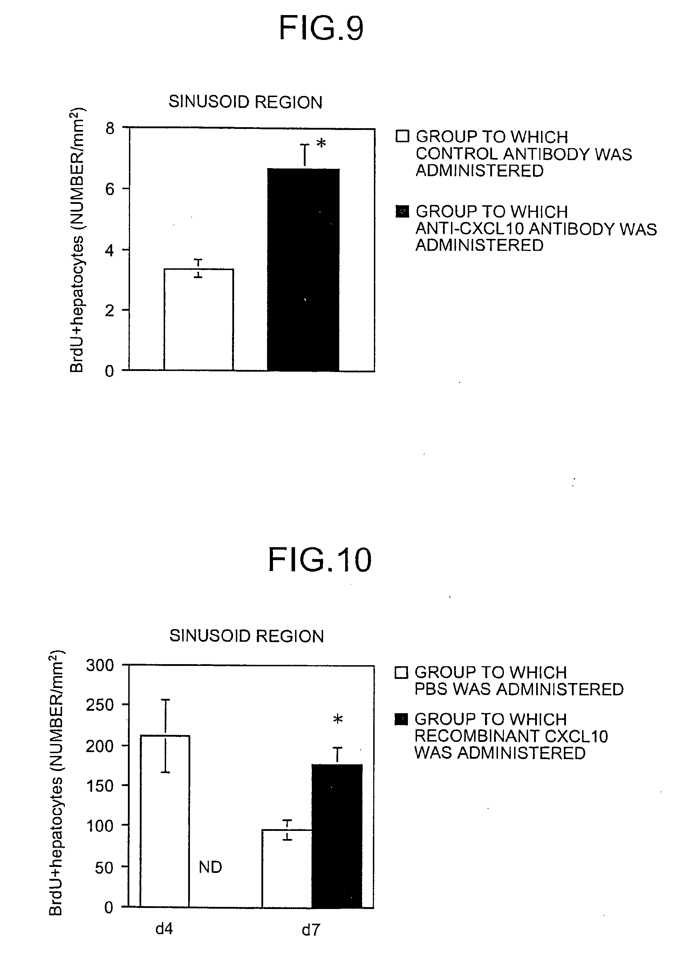

The results are shown in FIG. 9. The vertical axis (BrdU + hepatocytes [number/mm²]) in FIG. 9 represents the number of cells doubly stained per unit area in the sinusoid region. In FIG. 9, "*" indicates that a significant difference at p<0.05 by t-test was observed.
The vertical axis represents the number of immunologically stained portions per unit area.

Typically, the BrdU positive hepatocytes are scarcely observed in the sinusoid region in the normal mouse (BrdU labeling index (labeling index, see Example 8); 2.0% in the total hepatocytes). However, the number of the BrdU positive cells was significantly increased by treating with anti-CXCL10 antibody (BrdU labeling index; 4.1% in the total hepatocytes).

### (Example 7) Effects of CXCL10 on liver in normal mouse

Subsequently, it was examined whether the CXCL10 protein conversely inhibits the proliferation and the replication of the hepatocytes or not. BrdU (50 µg of BrdU suspended in 10 µL of sterile PBS) was once subcutaneously injected into neonatal mice on the 4th day after the birth. The mice were sacrificed one hour thereafter and on the 7th day (3 days after administering BrdU), the second lobe of the liver was collected and the immunostaining was given to the frozen sections. The recombinant CXCL10 protein (2 mg/kg, supplied from R & D system) or PBS was subcutaneously injected on the 3rd and 4th days after the birth.

The results are shown in FIG. 10. The vertical axis (BrdU + hepatocytes [number/mm²]) in FIG. 10 represents the number of cells doubly stained per unit area. In FIG. 10, "*" indicates that a significant difference at p<0.05 by t-test was observed. In the control group to which PBS was administered, many BrdU labeled cells were observed one hour after the BrdU administration (4 days after the birth, d4), but the number of the BrdU labeled cells was drastically decreased on the 3 day after the administration (7 days after the birth, d7). On the contrary, in the group to which the recombinant CXCL10 protein was administered, the BrdU labeled hepatocytes significantly remained on the 3 day after the administration (d7). Typically, BrdU labeling is rapidly attenuated along with postnatal division of hepatocytes. Therefore, it is speculated that the administration of CXCL10 inhibited the postnatal division of the hepatocytes and as its consequence the BrdU labeled hepatocytes significantly remained. That is, this result indicates that CXCL10 actually inhibits the division of the hepatocytes *in vivo.*

### (Example 8) Effects of CXCL10 and anti-CXCL10 antibody on proliferation of human hepatic cell line

Subsequently, human hepatic cell line, HepG2 cells (obtained from ATCC, 2 x 10⁴ cells) were cultured with the addition of the human recombinant CXCL10 protein (supplied from R & D system), the anti-human CXCL10 antibody (supplied from R & D system) or the control antibody in a 96-well flat bottom plate (supplied from Nunc) for 3 days.
After the culturing, 20 µL/well of WST-1 solution (supplied from Takara) was added thereto. One hour thereafter, the proliferation of the hepatic cells was assayed by measuring absorbance at 550 nm in each well. RNA was also extracted from the hepatic cells in the same manner as in the method in Example 1, and the expression of CXCR3-A and CXCR3-B which were the receptors for CXCL10 was examined by RT-PCR (30 cycles at 94°C for 45 seconds, 58°C for 45 seconds and 72°C for 60 seconds).

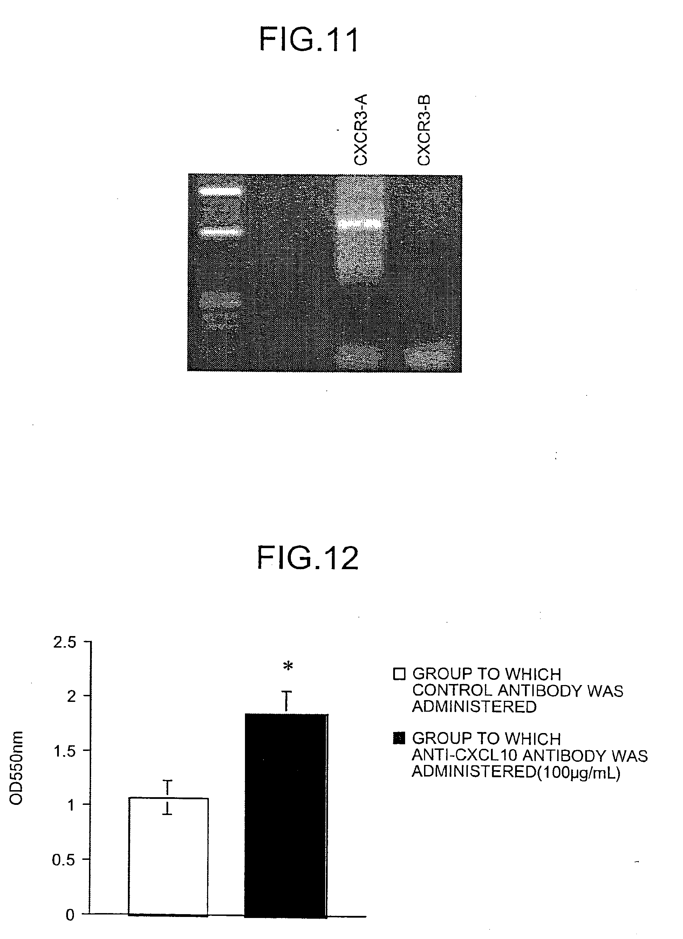

In the human hepatic cell line HepG2 cells cultured with CXCL10, CXCR3-A was strongly expressed, but no expression of CXCR3-B was observed (FIG. 11). That is, this indicates that CXCL10 exerts its functions through CXCR3-A.

The results of measuring the absorbance are shown in FIG. 12. In FIG. 12, the absorbance is described as mean ± SD. In FIG. 12, "*" indicates that a significant difference at p<0.05 by t-test was observed. The anti-CXCL10 antibody at the concentration administered to the mouse significantly promoted the proliferation of the human hepatic cell line HepG2 cells. These results indicate that CXCL10 at the high concentration also directly controls the division and proliferation of the human hepatocytes in vitro.

### (Example 9) Effects of anti-CXCL10 antibody on C57BL6J/JcL type II diabetes (NIDDM) model mouse induced with streptozotocin: Glucose tolerance test

C57BL6J/JcL mice (supplied from CLEA Japan Inc.) on the 14th day of pregnancy were bred and delivered. Streptozotocin (10 mg/mL, supplied from Sigma) at 20 µL/head was subcutaneously injected into C57BL6J/JcL mice (female, supplied from CLEA Japan Inc.) on the 2 day after the birth. The mice were bred until 4 weeks of age by giving CE-2 food (supplied from CLEA Japan Inc.) and sterile water, and bred for 2 weeks after 4 weeks of age by giving high fat diet (supplied from CLEA Japan Inc.) and sterile water. At the second week, phosphate buffer containing 100 µg/100 µL of the anti-CXCL10 antibody or phosphate buffer containing 100 µg/100 µL of the control antibody was intraperitoneally administered twice a week (one shot/week) for two weeks. On the 14th day, experiments to examine the effect of the antibody were performed.

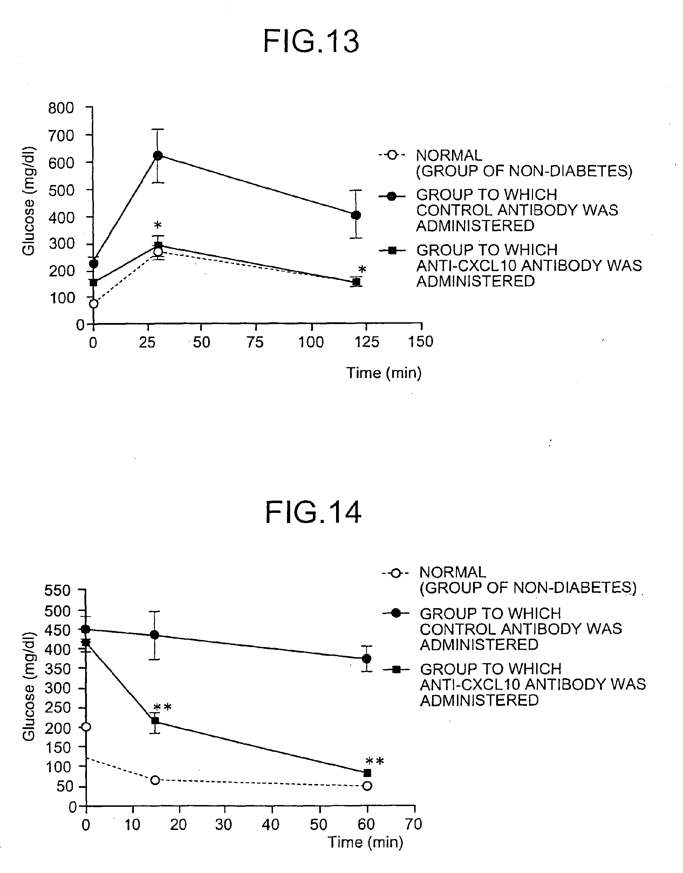

The mice were starved for 15 hours from the 13th day of the experiment, and on the 14th day, D-glucose (2 g/kg, supplied from Sigma) was intraperitoneally injected. Orbital blood was collected before the injection (0 minute), 30 minutes and 120 minutes after the injection, and blood sugar levels were measured using Glutest Ace blood sugar measuring device (supplied from Bombyx Medicine Co., Ltd.).
The results are shown in FIG. 13. FIG. 13 is a graph showing the changes of the blood sugar levels 0 minutes, 30 minutes and 120 minutes after glucose loading in normal mice (untreated), type II diabetes (NIDDM) model mice treated with the anti-CXCL10 antibody or the control antibody. In the figure, the vertical axis represents the blood sugar level (mg/dL) and the horizontal axis represents a measured time (minutes) after the loading. In FIG. 13, "*" indicates that a significant difference at p<0.05 by t-test was observed.

In the group to which the control antibody was administered, the blood sugar levels 30 minutes and 120 minutes after glucose loading were remarkably increased to 620 ± 168 mg/dL (n=5) and 405 ± 156 mg/dL (n=5), respectively. In untreated C57BL6J/JcL mice (supplied from CLEA Japan Inc.) aged 10 weeks, the levels were 270 ± 36 mg/dL (n=5) and 147 ± 21 mg/dL (n=5), respectively, and these are the normal range. Thus, it was shown that the mice in the control group in this experimental model caused the abnormal glucose tolerance. Meanwhile, in the group to which the anti-CXC110 antibody was administered, the levels remained at 284 ± 80 mg/dL (n=5) and 148 ± 43 mg/dL (n=5), respectively. These were the significantly low values compared with the control group (significant difference, p=0.017 and p=0.026, respectively by t-test). Therefore, it was found that the abnormal glucose tolerance in the NIDDM model mice was significantly ameliorated by administering the anti-CXCL10 antibody.

### (Example 10) Effects of anti-CXCL10 antibody on C57BL6J/JcL type II diabetes (NIDDM) model mouse induced with streptozotocin: Insulin resistance test

The NIDDM model mice were produced in the same way as in Example 9, and the insulin resistance test was performed. In the breeding condition in Example 9, without starving, human crystalline insulin (0.75 U/kg) was intraperitoneally administered on the 14th day of the experiment. The blood sugar levels after 0 minute (before the administration of insulin), 15 minutes and 60 minutes after the administration were measured using Glutest Ace blood sugar measuring device (supplied from Bombyx Medicine Co., Ltd.). The results are shown in FIG. 14. FIG. 14 is the graph showing the changes of the blood sugar levels 0 minutes, 15 minutes and 60 minutes after insulin loading in normal mice (untreated), type II diabetes (NIDDM) model mice treated with the anti-CXCL10 antibody or the control antibody. In the figure, the vertical axis represents the blood sugar level (mg/dL) and the horizontal axis represents the measured time (minutes) after the loading. In FIG. 14, "*" indicates that a significant difference at p<0.01 by t-test was observed.

In untreated C57BL6J/JcL mice (supplied from CLEA Japan Inc.) aged 10 weeks, the values after 0, 15 and 60 minutes were 125 ± 12 mg/dL (n=5), 63.2 ± 8.5 mg/dL (n=5) and 47.8 ± 7.7 mg/dL (n=5), respectively, and the blood sugar level was decreased well in response to the administration of insulin. In the group to which the control antibody was administered, the values were 448.2 ± 72 mg/dL (n=5), 433.8 ± 138 mg/dL (n=5) and 371.8 ± 72 mg/dL (n=5), respectively. Not only high blood sugar levels were shown before the administration of insulin but also the reactivity to insulin was low, and the blood sugar levels remained high. That is, this NIDDM model was determined to be less sensitive to insulin, i.e., exhibit the insulin resistance. On the contrary, in the group to which anti-CXCL10 antibody was administered, the blood sugar levels were 409.3 ± 35 mg/dL (n=5), 210.6 ± 59 mg/dL (n=5) and 75 ± 16 mg/dL (n=5), respectively. The mice responded to the insulin administration well and the:blood sugar levels were decreased although the high blood sugar levels were exhibited before the insulin administration. Compared with the control group, the blood sugar levels after the insulin administration in this group were decreased with significant difference (p=0.31, p=0.005, p=0.0005, respectively, t-test). Therefore, it has been demonstrated that the anti-CXCL10 antibody has the effect to ameliorate the insulin resistance.

### (Example 11) Insulin uptake capacity through hepatic insulin receptor in C57BL6J/JcL type II diabetes (NIDDM) model mouse induced with streptozotocin

C57BL6J/JcL mice (supplied from CLEA Japan Inc.) on the 14th day of pregnancy were bred and delivered. Streptozotocin (10 mg/mL, supplied from Sigma) at 20 µL/head was subcutaneously injected into C57BL6J/JcL mice (female, supplied from CLEA Japan Inc.) on the second day after the birth. The mice were bred until 4 weeks of age by giving CE-2 food (supplied from CLEA Japan Inc.) and sterile water, and bred for 2 weeks after 4 weeks of age by giving high fat diet (supplied from CLEA Japan Inc.) and sterile water. At the second week, phosphate buffer containing 100 µg/100 µL of the anti-CXCL10 antibody or phosphate buffer containing 100 µg/100 µL of the control antibody was intraperitoneally administered. Two hours after the administration, FITC-labeled insulin (0.75 U/kg, supplied from Molecular Probe) was intraperitoneally administered for the purpose of analyzing whether the insulin receptor in the liver became functional or not. After 30 minutes, the frozen sections of the liver were prepared based on the method in Example 1.

Each section was observed under the microscope. The binding of insulin was remarkably increased in the liver from the group to which the anti-CXCL10 antibody was administered, whereas the binding of insulin was scarcely observed in the liver from the group to which the control antibody was administered. The binding capacity of insulin in this Example appears to exhibit the function of the insulin receptor. Therefore, it is concluded that the uptake of insulin in the liver was increased and the reactivity/sensitivity to insulin was clearly ameliorated by administering the anti-CXCL10 antibody.

### (Example 12) Intracellular migration of β-catenin in hepatocytes in C57BL6J/JcL type II diabetes (NIDDM) model mouse induced with streptozotocin

C57BL6J/JcL mice (supplied from CLEA Japan Inc.) on the 14th day of pregnancy were bred and delivered. Streptozotocin (10 mg/mL, supplied from Sigma) at 20 µL/head was subcutaneously injected into C57BL6J/JcL mice (female, supplied from CLEA Japan Inc.) on the second day after the birth. The mice were bred until 4 weeks of age by giving CE-2 food (supplied from CLEA Japan Inc.) and sterile water, and bred for 2 weeks after 4 weeks of age by giving high fat diet (supplied from CLEA Japan Inc.) and sterile water. At the second week, phosphate buffer containing 100 µg/100 µL of the anti-CXCL10 antibody or phosphate buffer containing 100 µg/100 µL of the control antibody was intraperitoneally administered. Two hours after the administration, the frozen sections of the liver were prepared based on the method in Example 1, and analyzed by fluorescence double immunostaining in the same way as in Example 5. In the fluorescence double immunostaining, anti-β-catenin antibody (supplied from R & D) and anti-collagen type IV antibody were used as the primary antibody. Detection as blue and red fluorescence was performed using Alexa-647 labeled anti-rabbit IgG (supplied from Molecular Probe) and Alexa-564 labeled anti-rabbit IgG (supplied from Molecular Probe), respectively. Conditions other than the aforementioned were the same as in Example 5.

Each section after the immunostaining was observed under the microscope. β-Catenin which had migrated in cytoplasm and partial cell nuclei was observed in the liver from the group to which anti-CXCL10 antibody was administered, whereas β-catenin was detected in hepatocyte membrane in the control group. This indicates that β-catenin in the hepatocyte membrane migrates into the nucleus by administering the anti-CXCL10 antibody. That is, a novel and unexpected mechanism that the anti-CXCL10 antibody controls an intracellular signal of β-catenin was found as one of the mechanisms of hepatocyte replication by the anti-CXCL10 antibody.

### (Example 13) Augmentation of c-Myc expression in hepatocytes in C57BL6J/JcL type II diabetes (NIDDM) model mouse induced with streptozotocin

C57BL6J/JcL mice (supplied from CLEA Japan Inc.) on the 14th day of pregnancy were bred and delivered. Streptozotocin (10 mg/mL, supplied from Sigma) at 20 µL/head was subcutaneously injected into C57BL6J/JcL mice (female, supplied from CLEA Japan Inc.) on the second day after the birth. The mice were bred until 4 weeks of age by giving CE-2 food (supplied from CLEA Japan Inc.) and sterile water, and bred for 2 weeks after 4 weeks of age by giving high fat diet (supplied from CLEA Japan Inc.) and sterile water. At the second week, phosphate buffer containing 100 µg/100 µL of the anti-CXCL10 antibody or phosphate buffer containing 100 µg/100 µL of the control antibody was intraperitoneally administered. Two hours after the administration, the frozen sections of the liver were prepared based on the method in Example 1, and analyzed by immunostaining in the same way as in Example 5. The immunostaining was performed in the same way as in Example 5, except that an anti-phosphorylated c-Myc antibody (supplied from BioVision) was used as the primary antibody. Detection as blue color was performed with anti-rabbit IgG.

Each section after the immunostaining was observed under the microscope. Phosphorylated c-Myc was observed in the liver from the group to which the anti-CXCL10 antibody was administered whereas phosphorylation of c-Myc was scarcely observed in the liver from the group to which the control antibody was administered. This indicates that phosphorylation of c-Myc occurs along with intranuclear migration of β-catenin by the anti-CXCL10 antibody. It has been reported that the expression of c-Myc in the liver promotes the amelioration of sugar metabolism (Vaeva A et al., FASEB J.9:1067-1078,1995; and Riu E et al., FASEB J.fj.02-1163fje. Published online July 18, 2003), and it has been found that the anti-CXCL10 antibody plays the role for ameliorating the sugar metabolism in the liver also in this mechanism.

### INDUSTRIAL APPLICABILITY

The present invention is useful for proliferating the hepatocytes. For example, the present invention is useful when it is desirable to proliferate hepatocytes because of various hepatic diseases, and is also useful when regeneration of the hepatic tissue is intended because of hepatic tissue transplantation.
The present invention is also useful for ameliorating the insulin resistance. For example, the present invention is useful when the reactivity and sensitivity to insulin in the liver are augmented and the abnormal glucose tolerance is ameliorated in the patients exhibiting the glucose resistance, e.g., the patients with type II diabetes (NIDDM) and metabolic syndrome.

## Claims

1. A hepatocyte replication promoter comprising a neutralizing agent for CXCL10 which is one of chemokines as an active component.

2. The hepatocyte replication promoter according to claim 1 wherein the replication of at least mature hepatocytes is promoted.

3. The hepatocyte replication promoter according to claim 1 or 2 wherein the replication of the hepatocytes in impaired hepatic tissue is promoted.

4. The hepatocyte replication promoter according to any one of claims 1 to 3 wherein said impairment is selected from the group consisting of an impairment caused by a toxic substance, an impairment by a physical damage and an impairment caused by a pathogen.

5. The hepatocyte replication promoter according to claim 4 wherein said impairment caused by the toxic substance is an impairment caused by a disease selected from the group consisting of alcohol induced hepatitis and drug induced hepatic disorders.

6. The hepatocyte replication promoter according to any one of claims 1 to 3 wherein said pathogen is hepatitis virus.

7. The hepatocyte replication promoter according to claim 4 wherein said impairment by the physical damage is selected from the group consisting of liver transplantation, cell transplantation and surgical partial hepatectomy.

8. The hepatocyte replication promoter according to claim 4 wherein said impairment is fatty hepatitis.

9. The hepatocyte replication promoter according to any one of claims 1 to 8 wherein said neutralizing agent for CXCL10 is a factor which is specifically bound to CXCL10 and inhibits an activity of CXCL10.

10. The hepatocyte replication promoter according to claim 9 wherein said factor which inhibits the activity is an anti-CXCL10 antibody.

11. The hepatocyte replication promoter according to any one of claims 1 to 8 wherein said neutralizing agent for CXCL10 is a factor which inhibits expression of CXCL10.

12. The hepatocyte replication promoter according to any one of claims 1 to 8 wherein said neutralizing agent for CXCL10 is an antagonist of a receptor for CXCL10.

13. Use of a neutralizing agent for CXCL10 which is one of chemokines for producing a hepatocyte replication promoter.

14. A method for promoting proliferation of hepatocytes comprising a step of administering to the hepatocytes an effective amount of a neutralizing agent for CXCL10 which is one of chemokines.

15. An insulin resistance ameliorating agent comprising a neutralizing agent for CXCL10 which is one of chemokines as an active component.

16. The insulin resistance ameliorating agent according to claim 15 wherein said agent is capable of ameliorating insulin resistance in metabolic syndrome.

17. The insulin resistance ameliorating agent according to claim 15 wherein said agent is capable of ameliorating insulin resistance in type II diabetes.

18. The insulin resistance ameliorating agent according to any one of claims 15 to 17 wherein said neutralizing agent for CXCL10 is a factor which is specifically bound to CXCL10 and inhibits an activity of CXCL10.

19. The insulin resistance ameliorating agent according to claim 15 wherein said factor which inhibits the activity is an anti-CXCL10 antibody.

20. The insulin resistance ameliorating agent according to any one of claims 15 to 17 wherein said neutralizing agent for CXCL10 is a factor which inhibits expression of CXCL10.

21. The insulin resistance ameliorating agent according to any one of claims 15 to 17 wherein said neutralizing agent for CXCL10 is an antagonist of a receptor for CXCL10.

22. Use of a neutralizing agent for CXCL10 which is one of chemokines for producing an insulin resistance ameliorating agent.
